# EUROPÄISCHE PATENTSCHRIFT

(11) **EP 2 130 485 B1**
(45) Veröffentlichungstag und Bekanntmachung des Hinweises auf die Patenterteilung: **09.09.2015**
(21) Anmeldenummer: 09005616.9
(22) Anmeldetag: 22.04.2009
(51) Int. Cl.: A61B 1/12

(54) **Medizinisches Instrument**
Medical instrument
Instrument médical

(30) Priorität: 03.06.2008 DE 102008026457
(43) Veröffentlichungstag der Anmeldung: 09.12.2009
(73) Patentinhaber: Karl Storz GmbH & Co. KG, 78532 Tuttlingen (DE)
(72) Erfinder: Bettochi, Stefano, Prof., I-70124 Bari (IT)
(74) Vertreter: Hofmeister, Frank

(56) Entgegenhaltungen:
- EP-A- 1 800 596
- EP-A1- 1 726 254
- CN-Y- 2 534 688
- DE-A1- 19 805 532
- DE-U1- 20 011 409
- JP-A- 2007 252 834
- US-A- 5 133 336
- US-A1- 2006 287 576
- US-A1- 2007 186 660

## Beschreibung

Die Erfindung betrifft ein medizinisches Instrument mit Handhabe für endoskopische Eingriffe mit einem hohlen Schaft, wobei im Schaft mindestens zwei Kanäle ausgebildet sind und die Kanäle proximalseitig mit Zuführkanälen koppelbar sind.

Endoskopische Instrumente mit mindestens einem in der Handhabe ausgebildeten Kanal sind in verschiedenen Ausführungsformen bekannt. Die Kanäle sind beispielsweise als Saug-, Spül- und Lichtleiterkanäle ausgebildet, wobei die Saug- und Spülkanäle dazu dienen, das Operationsgebiet und die Endoskopoptik zu reinigen und der Lichtleiterkanal dazu verwendet wird, über Lichtleiter ausreichend Licht für die endoskopischen Untersuchungen und Eingriffe in das Operationsgebiet zu leiten.

Um beim Verbinden der Kanäle der Handhabe mit den externen Zuführkanälen sicherzustellen, dass immer die richtigen Kanäle und Zuführkanäle miteinander gekoppelt werden, ist es aus der Praxis bekannt, die Kanäle/Zuführkanäle mit einander entsprechenden Farbcodierungen, Pfeilen und/oder anderen Markierungen zu versehen. Diese Art der Anschlusskennung stellt aber gerade im oft hektischen Klinikbetrieb keinen Ausreichenden Schutz gegen Fehlanschlüsse der Kanäle dar.

Aus der US 5 518 501 A ist ein medizinisches Instrument bekannt, bei dem die mit den Kanäle der Handhabe zu koppelnden Zuführkanäle in einem Adapter zusammengefasst sind, der seinerseits an der Handhabe festlegbar ist. Bei diesem bekannten medizinischen Instrument ist der Adapter als an die Form der Handhabe angepasstes Bauteil ausgebildet, das nur mit dieser speziellen Handhabenform koppelbar ist. Zwar gewährleistet diese Ausgestaltung einen sehr hohen Fehlanschlussschutz, jedoch ist der konstruktive Aufwand sehr hoch und bedarf jeweils einer ganz bestimmten Handhabenform. Weitere Kupplungsmechanismen werden beschrieben in DE 19805532 A1, EP 1800 596 A1 und US 2007/0186660 A1.

Davon ausgehend liegt der Erfindung die **Aufgabe** zugrunde, ein medizinisches Instrument der eingangs genannten Art zu schaffen, das bei einfachem konstruktivem Aufbau eine hohe Anschlusssicherheit gewährleistet.

Die **Lösung** dieser Aufgabenstellung ist erfindungsgemäß dadurch gekennzeichnet, dass das proximale Ende mindestens eines ersten im Schaft angeordneten Kanals und das distale Ende zumindest eines mit diesem ersten Kanal zu koppelnden Zuführkanals hinsichtlich ihrer konstruktiven geometrischen Ausgestaltung so ausgebildet sind, dass dieser erste Kanal und der entsprechende Zuführkanal ausschließlich miteinander koppelbar sind.

Durch die individuelle konstruktiv geometrische Ausgestaltung der miteinander zu verbindenden proximalen und distalen Enden der im Schaft angeordneten Kanäle und der Zuführkanäle wird ein Höchstmaß an Anschlusssicherheit erzielt, da immer nur konstruktiv gleich aufgebaute Kanäle und Zuführkanäle miteinander koppelbar sind. Das Fehlanschließen ist bei der erfindungsgemäßen Ausgestaltung ausgeschlossen, da nicht zueinander passende Kanäle und Zuführkanäle nicht miteinander verbindbar sind.

Es ist bei diesem medizinischen Instrument ausreichend, nur die proximalen Enden der im Schaft angeordneten Kanäle sowie die distalen Enden der Zuführkanäle konstruktiv geometrisch aneinander anzupassen, ohne das gesamte medizinische Instrument an die Zuführkanäle anpassen zu müssen.

Gemäß einer bevorzugten Ausführungsform der Erfindung wird vorgeschlagen, dass die proximalen Enden aller im medizinischen Instrument angeordneten Kanäle sowie die distalen Enden aller Zuführkanäle hinsichtlich ihrer konstruktiven geometrischen Ausgestaltung so ausgebildet sind, dass jeder Kanal des medizinischen Instruments ausschließlich mit dem entsprechend konstruktiv geometrisch ausgestalteten Zuführkanal koppelbar ist.

Um das Koppeln der instrumentensetigen Kanäle mit den Zuführkanälen zu erleichtern, wird mit einer praktischen Ausführungsform der Erfindung vorgeschlagen, dass mehrere Zuführkanäle in einem mit dem medizinischen Instrument koppelbaren Anschlussadapter zusammengefasst sind, wobei der Anschlüssadapter vorteilhafterweise über einen Kupplungsmechanismus am medizinischen Instrument festlegbar ist.

Gemäß der Erfindung wird vorgeschlagen, dass der Kupplungsmechanismus als Rastverbindung, beispielsweise mit einem Rasthaken und einer entsprechenden Rastaufnahme für den Rasthaken, ausgebildet ist.

Zur Ausbildung der die Enden der Kanäle/Zuführkanäle individualisierenden Geometrie wird gemäß einer ersten erfindungsgemäßen Ausgestaltungsform vorgeschlagen, dass die Kanäle/Zuführkanäle im Bereich ihrer miteinander zu koppelnden Enden hinsichtlich ihrer Innen- oder Außendurchmesser und/oder ihrer Querschnittsform aneinander angepasst sind.

Zusätzlich oder alternativ zu der Anpassung der Querschnittsformen und/oder Durchmesser wird gemäß einer zweiten erfindungsgemäßen Ausgestaltungsform vorgeschlagen, dass die Kanäle des medizinischen Instruments sowie die in einem Anschlussadapter zusammengefassten Zuführkanäle im Bereich ihrer miteinander zu koppelnden Enden hinsichtlich ihrer Länge aneinander angepasst sind.

Schließlich wird mit der Erfindung vorgeschlagen, dass in mindestens einem Kanal des medizinischen Instruments oder in mindestens einem Zuführkanal ein Rückschlagventil oder Dosierventil angeordnet ist, um die Saug- und/oder Spülleistung variieren zu können und Fluidströmungen in die falsche Richtung ausschließen zu können.

Die Erfindung betrifft weiterhin eine Handhabe des medizinischen Instrument(s) für endoskopische Eingriffe, wobei in der den Schaft tragenden Handhabe mindestens zwei sich aus dem Schaft fortsetzende Kanäle ausgebildet sind und die in der Handhabe angeordneten Kanäle proximalseitig mit Zuführkanälen koppelbar sind.

Um Verwechslungen beim Koppeln der die in der Handhabe angeordneten Kanäle mit den Zuführkanälen zu verhindern, wird mit der Erfindung vorgeschlagen, dass das proximale Ende mindestens eines ersten in der Handhabe angeordneten Kanals und das distale Ende zumindest eines mit diesem ersten Kanal der Handhabe zu koppelnden Zuführkanals hinsichtlich ihrer konstruktiven geometrischen Ausgestaltung so ausgebildet sind, dass dieser erste Kanal und der entsprechende Zuführkanal ausschließlich miteinander koppelbar sind.

Um das Koppeln der handhabensetigen Kanäle mit den Zuführkanälen zu erleichtern, wird mit einer praktischen Ausführungsform der Erfindung vorgeschlagen, dass mehrere Zuführkanäle in einem mit der Handhabe koppelbaren Anschlussadapter zusammengefasst sind, wobei der Anschlussadapter vorteilhafterweise über einen Kupplungsmechanismus an der Handhabe festlegbar ist.

Gemäß der Erfindung wird vorgeschlagen, dass der Kupplungsmechanismus als Rastverbindung, beispielsweise mit einem Rasthaken und einer entsprechenden Rastaufnahme für den Rasthaken, ausgebildet ist.

Als Kanäle und Zuführkanäle im Sinne der Erfindung gelten neben den bekannten Saug- und/oder Spülkanälen auch Video-Anschlüsse und elektrische Anschlüsse, wie beispielsweise HF-Anschlüsse.

Weitere Merkmale und Vorteile der Erfindung ergeben sich anhand der zugehörigen Zeichnung, in der ein Ausführungsbeispiel eines erfindungsgemäßen medizinischen Instruments nur beispielhaft dargestellt ist, ohne die Erfindung auf dieses Ausführungsbeispiel zu beschränken. In der Zeichnung zeigt:
- Fig. 1: eine schematische Seitenansicht eines erfindungsgemäßen medizinischen Instruments im ungekoppelten Zustand;
- Fig. 2: einen Schnitt entlang der Linie II-II gemäß Fig. 1;
- Fig. 3: einen Ansicht des Details III gemäß Fig. 1, jedoch das medizinische Instrument mit angekoppelten Zuführkanälen darstellend;
- Fig. 4: einen Schnitt entlang der Linie IV-IV gemäß Fig. 3 und
- Fig. 5: eine vergrößerte Darstellung eines mit dem medizinischen Instrument zu koppelnden Anschlussadapters.

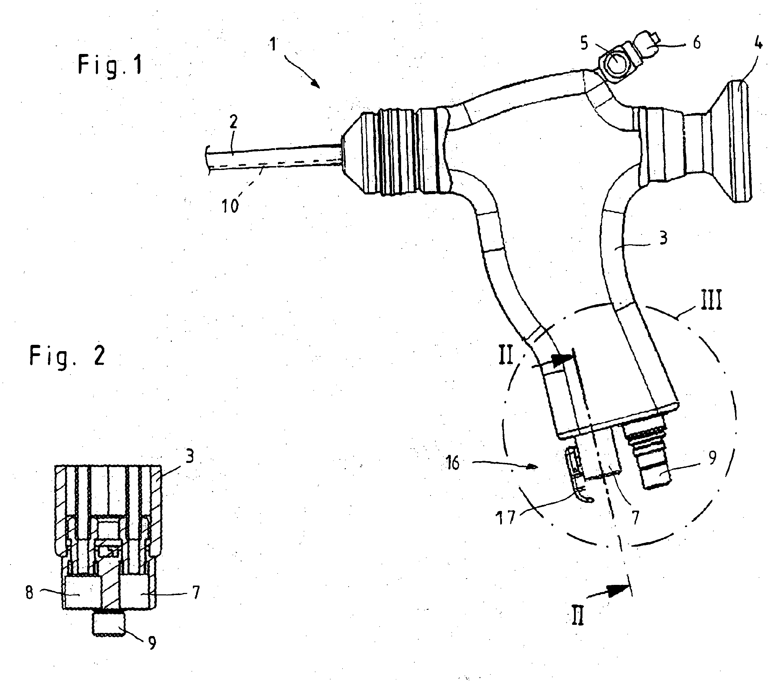

Die Abbildung Fig. 1 zeigt ein Endoskop 1, das im Wesentlichen aus einem hohlen Schaft 2 und einer den Schaft 2 tragenden Handhabe 3 besteht. Zur Untersuchung des Operationsraums sowie zum Führen der Instrumente während der Operation weist das Endoskop 1 ein aus verschiedenen Linsen aufgebautes optisches System auf, das proximal in einer Okulareinheit 4 endet.

Proximalseitig weist das Endoskop 1 in der in Fig. 1 dargestellten Seitenansicht darüber hinaus einen über ein Ventil 5 verschließbaren Zugang 6 zum hohlen Schaft 2 auf, über den medizinische Instrumente, wie beispielsweise Schneid- und/oder Greifinstrumente, in den hohlen Schaft 2 und schließlich in das Operationsgebiet einführbar sind. Das Ventil 5 dient dazu, bei nicht eingesetzten medizinischen Instrument den hohlen Schaft 2 fluiddicht zu verschließen, um beispielsweise bei der Laparoskopie ein Entweichen des zur Ausbildung des Pneumoperitoneums dienenden Gases zu verhindern.

Selbstverständlich ist es auch möglich, dass das Endoskop 1 mehr als nur einen Zugang 6 zum Einführen medizinischer Instrumente in den hohlen Schaft 2 aufweist.

Wie weiterhin aus Fig. 1 bis 4 ersichtlich, sind in der Handhabe 3 verschiedene Kanäle, nämlich ein Saugkanal 7, ein Spülkanal 8 sowie ein Lichtkanal 9, ausgebildet, die in den hohlen Schaft 2 übergehen. Während der Saugkanal 7 und der Spülkanal 8 in der Regel frei in den hohlen Schaft 2 übergehen, setzt sich der handhabenseitige Lichtkanal 9 in einem Lichtkanal 10 im Schaft 2 fort.

Selbstverständlich ist es auch möglich, den hohlen Schaft 2 so auszugestalten, dass die handhabenseitigen Saug- und Spülkanäle 7, 8 in separate Kanäle im Schaft 2 übergehen sowie mindestens ein separater Arbeitskanäle zur Aufnahme des/der über den Zugang 6 bzw. die Zugänge 6 in den hohlen Schaft 2 eingesetzten medizinischen Instruments/Instrumente im Schaft 2 ausgebildet ist. Ebenso können selbstverständlich mehr als nur drei Kanäle 7, 8 und 9 in der Handhabe 3 angeordnet sein.

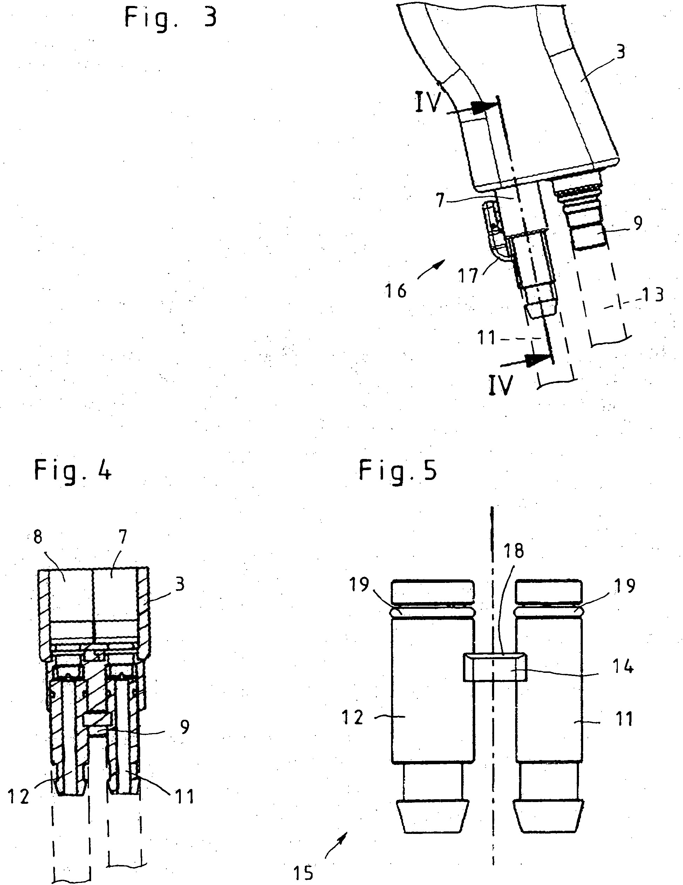

Zur Inbetriebnahme des Endoskops 1 sind an die in der Handhabe 3 angeordneten Kanäle 7, 8 und 9 von außen zugeführte Zuführkanäle 11, 12 und 13 anschließbar, wie dies in Fig. 3 und 4 dargestellt ist.

Als Kanäle und Zuführkanäle im Sinne des hier dargestellten und beschriebenen medizinischen Instruments gelten auch Video-Anschlüsse und elektrische Anschlüsse, wie beispielsweise HF-Anschlüsse.

Wie weiterhin aus Fig. 3 bis 5 ersichtlich, sind bei der dargestellten Ausführungsform der Saug-Zuführkanal 11 und der Spül-Zuführkanal 12 über einen Steg 14 zu einem Anschlussadapter 15 zusammengefasst, so dass die beiden Zuführkanäle 11 und 12 gleichzeitig als einstückiger Kupplungsblock an den Kanälen 7 und 8 der Handhabe 3 festgelegt werden können.

Bei der dargestellten Ausführungsform ist der Licht-Zuführkanal 13 als separater Zuführkanal 13 ausgebildet, jedoch ist es selbstverständlich auch möglich, alle Zuführkanäle 11, 12 und 13 in einem Anschlussadapter 15 zusammenzufassen.

Das Festlegen des Anschlussadapters 15 an den proximalen Enden der Kanäle 7 und 8 der Handhabe 3 erfolgt über einen Kupplungsmechanismus 16, der bei der dargestellten Ausführungsform als Rastverbindung mit einem an der Handhabe 3 gelagerten Rasthaken 17 und einer am Steg 14 ausgebildeten Rastaufnahme 18 für den Rasthaken 17 ausgebildet ist.

Bei der in Fig. 5 dargestellten Ausführungsform weisen die die Zuführkanäle 11 und 12 distalseitig als O-Ringe ausgebildete Dichtelemente 19 auf, um eine fluiddichte Ankopplung an die Kanäle 7 und 8 der Handhabe 3 zu gewährleisten. Selbstverständlich ist es auch möglich, die Dichtelemente 19 am proximalseitigen Ende der Kanäle 7 und 8 der Handhabe 3 anzuordnen.

Um zu gewährleisten, dass immer der richtige Zuführkanal 11, 12 oder 13 an den zugehörigen Kanal 7, 8 oder 9 der Handhabe 3 angeschlossen wird, also beispielsweise Saug-Zuführkanal 11 an den Saugkanal 7 der Handhabe 3 und Spül-Zuführkanal 12 an den Spülkanal 8 der Handhabe 3, ist das proximale Ende mindestens eines ersten in der Handhabe 3 angeordneten Kanals 7, 8 oder 9 und das distale Ende zumindest eines mit diesem ersten Kanal 7, 8 oder 9 der Handhabe 3 zu koppelnden Zuführkanals 11, 12 oder 13 hinsichtlich seiner konstruktiven geometrischen Ausgestaltung so ausgebildet, dass dieser erste Kanal 7, 8 oder 9 und der entsprechende Zuführkanal 11, 12 oder 13 ausschließlich miteinander koppelbar sind.

Eine solche konstruktiv geometrische Ausgestaltung kann beispielsweise so aussehen, dass das proximale Ende eines Kanals 7, 8 oder 9 dreieckig ausgebildet ist und das distale Ende des mit eben diesem Kanal 7, 8 oder 9 zu koppelnden Zuführkanals 11, 12 oder 13 ebenfalls dreieckig ausgebildet ist, um sicherzustellen, dass ausschließlich diese gegenseitig entsprechend ausgebildeten Kanäle und Zuführkanäle miteinander koppelbar sind.

Diese Individualisierung der proximalen Enden der handhabenseitigen Kanäle 7, 8 und/oder 9 sowie der distalen Enden der zugehörigen Zuführkanäle 11, 12 und/oder 13 stellt sicher, dass ausschließlich hinsichtlich der konstruktiv geometrischen Ausgestaltung übereinstimmende Kanäle 7, 8 und/oder 9 und Zuführkanäle 11, 12 und/oder 13 miteinander koppelbar sind, so dass ein Fehlanschließen aufgrund der konstruktiv nicht passenden Kontur des Gegenstücks auszuschließen ist.

Zur Ausbildung der die Enden der Kanäle/Zuführkanäle individualisierenden Geometrie wird gemäß einer ersten Ausgestaltungsform vorgeschlagen, dass die Kanäle/Zuführkanäle im Bereich ihrer miteinander zu koppelnden Enden hinsichtlich ihrer Innen- oder Außendurchmesser und/oder ihrer Querschnittsform aneinander angepasst sind.

Zusätzlich oder alternativ zu der Anpassung der Querschnittsformen und/oder Durchmesser wird gemäß einer zweiten Ausgestaltungsform vorgeschlagen, dass die Kanäle 7, 8 und 9 der Handhabe 3 sowie die in einem Anschlussadapter 15 zusammengefassten Zuführkanäle 11, 12 und 13 im Bereich ihrer miteinander zu koppelnden Enden hinsichtlich ihrer Länge aneinander angepasst sind.

Ein solchermaßen ausgebildetes Endoskop 1 zeichnet sich dadurch aus, dass bei einfachem konstruktivem Aufbau ein Höchstmaß an Anschlusssicherheit hinsichtlich der Verbindung der in der Handhabe 3 angeordneten Kanäle 7, 8 und 9 mit den zugehörigen Zuführkanälen 11, 12 und 13 gewährleistet ist.

**Bezugszeichenliste**

| | | | |
|---|---|---|---|
| 1 | Endoskop | 18 | Rastaufnahme |
| 2 | Schaft | 19 | Dichtelement |
| 3 | Handhabe | | |
| 4 | Okulareinheit | | |
| 5 | Ventil | | |
| 6 | Zugang | | |
| 7 | Saugkanal | | |
| 8 | Spülkanal | | |
| 9 | Lichtkanal | | |
| 10 | Lichtkanal | | |
| 11 | Saug-Zuführkanal | | |
| 12 | Spül-Zuführkanal | | |
| 13 | Licht-Zuführkanal | | |
| 14 | Steg | | |
| 15 | Anschlussadapter | | |
| 16 | Kupplungsmechanismus | | |
| 17 | Rasthaken | | |

## Patentansprüche

1. Medizinisches Instrument für endoskopische Eingriffe mit einer Handhabe (3) und einen hohlen Schaft (2), wobei in der den Schaft (2) tragenden Handhabe (3) mindestens zwei sich aus dem Schaft (2) fortsetzende Kanäle (7, 8, 9) ausgebildet sind und die Kanäle (7, 8, 9) proximalseitig mit Zuführkanälen (11, 12, 13) koppelbar sind, wobei das proximale Ende mindestens eines ersten in der Handhabe (3) angeordneten Kanals (7, 8 oder 9) und das distale Ende zumindest eines mit diesem ersten Kanal (7, 8 oder 9) der Handhabe (3) zu koppelnden Zuführkanals (11, 12 oder 13) hinsichtlich ihrer konstruktiven geometrischen Ausgestaltung so ausgebildet sind, dass dieser erste Kanal (7, 8 oder 9) und der entsprechende Zuführkanal (11, 12 oder 13) ausschließlich miteinander koppelbar sind,
**dadurch gekennzeichnet,**
**dass** mindestens zwei Zuführkanäle (11, 12) über einen die Zuführkanäle (11, 12) miteinander verbindenden Steg (14) zu einem mit dem medizinischen Instrument. koppelbaren Anschlussadapter (15) zusammengefasst sind und, dass der Anschlussadapter (15) über einen als Rastverbindung mit einem Rasthaken (17) und einer Rastaufnahme (18) ausgebildeten Kupplungsmechanismus an der Handhabe (3) festlegbar ist, wobei die Rastaufnahme am Steg ausgebildet ist.

2. Medizinisches Instrument nach Anspruch 1, **dadurch gekennzeichnet, dass** die proximalen Enden aller Kanäle (7, 8, 9) des medizinischen Instruments sowie die distalen Enden aller Zuführkanäle (11, 12, 13) hinsichtlich ihrer konstruktiven geometrischen Ausgestaltung so ausgebildet sind, dass jeder Kanal (7, 8, 9) ausschließlich mit den entsprechend konstruktiv geometrisch ausgestalteten Zuführkanal (11, 12, 13) koppelbar ist.

3. Medizinisches Instrument nach Anspruch 1 oder 2, **dadurch gekennzeichnet, dass** die Kanäle (7, 8, 9) des medizinischen Instruments und die Zuführkanäle (11, 12, 13) im Bereich ihrer miteinander zu koppelnden Enden hinsichtlich ihrer Innen- oder Außendurchmesser und/oder ihrer Querschnittsform aneinander angepasst sind.

4. Medizinisches Instrument nach einem der Ansprüche 1 bis 3, **dadurch gekennzeichnet, dass** die Kanäle (7 oder 8) des medizinischen Instruments sowie die in einem Anschlussadapter (15) zusammengefassten Zuführkanäle (11, 12, 13) im Bereich ihrer miteinander zu koppelnden Enden hinsichtlich ihrer Länge aneinander angepasst sind.

5. Medizinisches Instrument nach einem der Ansprüche 1 bis 4, **dadurch gekennzeichnet, dass** in mindestens einem Kanäle (7, 8, 9) des medizinischen Instruments bzw. einem Zuführkanal (11 oder 12) ein Rückschlagventil oder Dosierventil angeordnet ist.

6. Medizinisches Instrument nach einem der Ansprüche 1 bis 4, **dadurch gekennzeichnet, dass** mindestens ein Kanal und ein Zuführkanal als Kanal für Video-Anschlüsse oder elektrische Anschlüsse, wie beispielsweise HF-Anschlüsse, ausgebildet ist.

## Claims

1. A medical instrument for endoscopic procedures, having a handle (3) and a hollow shaft (2), wherein in the handle (3) that supports the shaft (2) at least two channels (7, 8, 9) are configured extending from the shaft (2) and the channels (7, 8, 9) can be coupled on the proximal end with supply conduits (11, 12, 13), wherein the proximal end of at least one first channel (7, 8, 9) positioned in the handle (3) and the distal end of at least one supply conduit (11, 12 or 13) that is to be coupled with this first channel (7, 8 or 9) of the handle (3) are configured with respect to their geometric structural design in such a way that this first channel (7, 8 or 9) and the corresponding supply conduit (11, 12 or 13) can be coupled only with one another,
**characterized in that**
at least two supply conduits (11, 12) are combined via a stud (14) connecting the supply conduits (11, 12) to one another in a connecting adapter (15) that may be coupled to the medical instrument, and that the connecting adapter (15) may be secured to the handle (3) by means of a coupling mechanism configured as a catch connection having a catch hook (17) and a catch recess (18), wherein the catch recess is formed on the stud.

2. The medical instrument according to Claim 1, **characterized in that** the proximal ends of all channels (7, 8, 9) of the medical instrument and the distal ends of all supply conduits (11, 12, 13) are configured with respect to their geometric structural design in such a way that every channel (7, 8, 9) can be coupled only with the correspondingly constructed geometrically designed supply conduit (11, 12, 13).

3. The medical instrument according to Claim 1 or 2, **characterized in that** the channels (7, 8, 9) of the medical instrument and the supply conduits (11, 12, 13) in the area of their ends that are to be coupled are adapted to one another with respect to their inner diameters or outer diameters and/or their cross-sectional form.

4. The medical instrument according to one of Claims 1 through 3, **characterized in that** the channels (7 or 8) of the medical instrument, as well as the supply conduits (11, 12, 13) combined in a connecting adapter (15), are adapted to one another with respect to their length in the area of their ends that are to be mutually coupled.

5. The medical instrument according to one of Claims 1 through 4, **characterized in that** a check valve or a dosing valve is positioned in at least one channel (7, 8, 9) of the medical instrument or at least one supply conduit (11 or 12).

6. The medical instrument according to one of Claims 1 through 4, **characterized in that** at least one channel and one supply conduit is configured as a channel for video connections or electrical connections, such as HF connections.

## Revendications

1. Instrument médical destiné à des interventions endoscopiques, comprenant une poignée (3) et un corps allongé creux (2), instrument
dans lequel à l'intérieur de la poignée (3) portant le corps allongé creux (2), sont formés au moins deux canaux (7, 8, 9) en prolongement du corps allongé (2), et les canaux (7, 8, 9) peuvent être couplés, côté proximal, à des canaux d'alimentation (11, 12, 13),
et dans lequel l'extrémité proximale d'au moins un premier canal (7, 8 ou 9) agencé dans la poignée (3), et l'extrémité distale d'au moins un canal d'alimentation (11, 12 ou 13) à coupler à ce premier canal (7, 8 ou 9) de la poignée (3), sont réalisées de façon telle, quant à leur configuration géométrique de construction, que ce premier canal (7, 8 ou 9) et le canal d'alimentation (11, 12 ou 13) correspondant puissent être couplés exclusivement l'un à l'autre,
**caractérisé**
**en ce qu'**au moins deux canaux d'alimentation (11, 12) sont regroupés, par l'intermédiaire d'une branche de liaison (14) reliant l'un à l'autre les deux canaux d'alimentation (11, 12), en un adaptateur de raccordement (15) pouvant être couplé à l'instrument médical, et en ce que l'adaptateur de raccordement (15) peut être fixé à la poignée (3) par l'intermédiaire d'un mécanisme de couplage réalisé en tant que liaison par encliquetage avec un crochet d'encliquetage (17) et un logement d'encliquetage (18), ce logement d'encliquetage (18) étant formé sur la branche de liaison (14).

2. Instrument médical selon la revendication 1, **caractérisé en ce que** les extrémités proximales de tous les canaux (7, 8, 9) de l'instrument médical, ainsi que les extrémités distales de tous les canaux d'alimentation (11, 12, 13) sont réalisées, quant à leur configuration géométrique de construction, de façon à ce que chaque canal (7, 8, 9) puisse exclusivement être couplé avec le canal d'alimentation (11, 12, 13) présentant une configuration géométrique de construction correspondante.

3. Instrument médical selon la revendication 1 ou la revendication 2, **caractérisé en ce que** les canaux (7, 8, 9) de l'instrument médical et les canaux d'alimentation (11, 12, 13) sont, dans la zone de leurs extrémités à coupler mutuellement, adaptés les uns aux autres quant à leurs diamètres intérieur ou extérieur et/ou quant à leur forme de section transversale.

4. Instrument médical selon l'une des revendications 1 à 3, **caractérisé en ce que** les canaux (7 ou 8) de l'instrument médical, ainsi que les canaux d'alimentation (11, 12, 13) regroupés en un adaptateur de raccordement (15), sont adaptés les uns aux autres quant à leur longueur, dans la zone de leurs extrémités à coupler mutuellement.

5. Instrument médical selon l'une des revendications 1 à 4, **caractérisé en ce que** dans au moins un des canaux (7, 8, 9) de l'instrument médical ou dans un canal d'alimentation (11 ou 12), est agencé un clapet anti-retour ou une vanne de dosage.

6. Instrument médical selon l'une des revendications 1 à 4, **caractérisé en ce qu'**au moins un canal et un canal d'alimentation est réalisé en tant que canal pour des branchements vidéo ou des branchements électriques, comme par exemple des branchements HF.
